# EUROPEAN PATENT APPLICATION

(11) **EP 4 782 027 A1**
(43) Date of publication of application: **29.07.2026**
(21) Application number: 26153261.8
(22) Date of filing: 21.01.2026
(51) Int. Cl.: A61M 1/36, A61B 5/0215, A61B 5/00, A61M 25/00

(54) **DEVICES AND METHODS FOR DETECTING MIXING CLOUD LOCATIONS**

(30) Priority: 22.01.2025 US 202563748197 P
(71) Applicant: Abiomed, Inc., Danvers, MA 01923 (US)
(72) Inventor: FANTUZZI, Glen, Danvers, 01923 (US)
(74) Representative: Wittmer, Maximilian

(57) **Abstract**

Devices and techniques for estimating/detecting a location of a mixing cloud in a vessel of a subject may be provided. The techniques may include positioning a first tubular member within the vessel and flowing blood through a first lumen from a proximal end to a distal end of either the first tubular member or a second tubular member in a direction retrograde to a direction blood is natively flowing in the vessel. The techniques may include determining a plurality of blood-related characteristics of blood flowing in the vessel, such as arterial blood oxygen saturation values, each determined from blood acquired at a different axial position along the first tubular member, and/or pressure values, each determined from a pressure sensor at different axial positions along an exterior surface of the first tubular member. The techniques may include estimating a location of the mixing cloud based on the blood-related characteristics.

## Description

### CROSS-REFERENCE TO RELATED APPLICATIONS

The present application claims priority to U.S. Provisional Patent Application No. 63/748,197, filed January 22, 2025, the contents of which are incorporated herein in its entirety.

### TECHNICAL FIELD

The present disclosure is drawn to the field of extracorporeal membrane oxygenation (ECMO), and specifically the determination of ECMO mixing cloud locations within a blood vessel.

### BACKGROUND

Various cardiac-related issues, such as acute cardiac heart failure, compromise central hemodynamics and consequently microvascular perfusion throughout the body. ECMO (including, e.g., veno-arterial ECMO, VA-ECMO) may be used as a bridge to recovery or to other destination therapy. That is, at some point, a patient must necessarily be weaned off ECMO support. However, traditional techniques for determining the level of a patient's recovery for effectively weaning the patient off ECMO support are lacking.

### BRIEF SUMMARY

Various deficiencies in the prior art are addressed below by the disclosed techniques and devices.

In various aspects, a method for detecting a location of a mixing cloud in a vessel (such as an aorta) of a subject may be provided. The method may include positioning a first tubular member within the vessel. The method may include flowing blood through a first lumen from the proximal end to the distal end of either the first tubular member or a second tubular member (e.g., a separate ECMO cannula) in a direction retrograde to a direction blood is natively flowing in the vessel. The method may include determining a plurality of blood-related characteristics of blood flowing in the vessel. The characteristics may include a plurality of arterial blood oxygen saturation values and/or a plurality of pressure values. Each saturation value may be determined from blood acquired at a different axial position along the first tubular member. Each pressure value may be determined from a pressure sensor of a plurality of pressure sensors positioned at or on different axial positions along an exterior surface of the first tubular member. The method may include estimating a location of the mixing cloud based on the blood-related characteristics.

The first tubular member or the second tubular member may include an extracorporeal membrane oxygenation (ECMO) cannula. The first tubular member may define a first plurality of lumens, where each lumen of the first plurality of lumens may extend from the proximal end to an external surface of the first tubular member at a different axial position along the first tubular member as compared to other lumens of the first plurality of lumens. Each lumen of the first plurality of lumens may be configured to transport blood in a proximal direction.

The method may include drawing a plurality of blood samples, each of which may be drawn through one or more lumens of the first plurality of lumens. The plurality of blood-related characteristics may be determined by measuring the arterial blood oxygen saturation values of each blood sample. In some embodiments, each blood sample may be drawn simultaneously. In some embodiments, each blood sample may be drawn sequentially. In some embodiments, each blood sample may be drawn at a time controlled by at least one processor.

The method may include providing a plurality of third tubular members, where each third tubular member may have a lumen extending therethrough. The method may include inserting one tubular member of the plurality of third tubular members at least partially into one of the first plurality of lumens, to allow blood to be drawn through the lumen of the third tubular member. The plurality of third tubular members may be operably coupled to a hub at or near a proximal end of each third tubular member.

Each lumen of the first plurality of lumens may include an electrical or optical communication conduit coupled to a pressure sensor of the plurality of pressure sensors. The method may include receiving information from the plurality of pressure sensors. The plurality of blood-related characteristics may be determined by determining a pressure value based on the received information from each pressure sensor. In some embodiments, each pressure sensor may be oriented in a non-orthogonal direction relative to the axis of the first tubular member. In some embodiments, all pressure sensors of the plurality of pressure sensors are oriented towards the distal end or the proximal end of the first tubular member.

The first tubular member may extend through a femoral artery, and may extend at least partially through an aortic arch.

In some embodiments, the location of the mixing cloud may be estimated by determining where oxygenated blood passing through the first lumen is mixing with unoxygenated blood from the subject's heart based on the oxygen content of the blood pulled from each location in the aorta. In some embodiments, the location of the mixing cloud may be estimated by determining a direction of flow at each pressure sensor location based on the plurality of pressure values and estimating a location of an inflection point in the direction of flow along the length of the first tubular member based on the determined directions of flow.

The method may include locking the first tubular member into a fixed position after positioning the first tubular member within the vessel. The method may include adjusting the position of the first tubular member after determining the plurality of blood-related characteristics of blood flowing in the vessel.

The method may include measuring an acceleration of the first tubular member. The plurality of blood-related characteristics may be determined based at least partially on the measured acceleration.

The method may include graphically displaying the estimated location of the mixing cloud. The method may include graphically displaying a location of the first tubular member and/or a blood pump.

The method may include receiving information from at least one sensor operably coupled to a blood pump positioned in a heart. In some embodiments, estimating the location of the mixing cloud may be based on the blood-related characteristics and the information received from the at least one sensor operably coupled to the blood pump. In some embodiments, the location of the mixing cloud may be estimated relative to a position of the blood pump.

In various aspects, a device for detecting a location of a mixing cloud in a vessel of a subject may be provided. The device may include a tubular member defining a first lumen extending from a proximal end to a distal end of the first tubular member. The device may include a first plurality of lumens, where each lumen of the first plurality of lumens extends from the proximal end to an external surface of the first tubular member at a different axial position along the first tubular member as compared to other lumens of the first plurality of lumens. Each lumen of the first plurality of lumens may be configured to allow blood to be drawn from a subject and through the proximal end of the first tubular member.

The device may include a plurality of pressure sensors, each pressure sensor being positioned at the distal end of one of the first plurality of lumens. In some embodiments, each of these pressure sensors may be oriented in a non-orthogonal direction relative to the axis of the first tubular member. In some embodiments, all of these pressure sensors are oriented towards the distal end or the proximal end of the first tubular member.

The first tubular member may have a first circumference at the proximal end, and a second circumference at the distal ends of each of the first plurality of lumens, the second circumference being greater than the first circumference.

The device may include at least one fluoroscopic marker and/or radiopaque material. This may include a metal band and/or a filler polymer placed on the first tubular member at predetermined locations.

In various aspects, a system may be provided. The system may include a device as disclosed herein, and a controller operably coupled to the device and at least one pump configured to cause blood to flow through the device.

The controller may include at least one processor configured to measure an arterial blood oxygen saturation value of each blood sample of a plurality of blood samples received from the device, correlate each arterial blood oxygen saturation value with an axial location along the first tubular member, and estimate a location of the mixing cloud by calculating an axial location at which a theoretical arterial blood oxygen saturation value, based on the measured arterial blood oxygen saturation values and the correlation, would be expected to equal a predetermined value or range of values.

The controller may include at least one processor configured to determine a pressure value associated with each pressure sensor reading received from a plurality of pressure sensors, correlate each pressure value with an axial location along the first tubular member, and estimate a location of the mixing cloud by calculating an axial location at which a theoretical pressure value, based on the determined pressure values and the correlation, would be expected to equal a predetermined value or range of values.

The system may include an extracorporeal membrane oxygenation (ECMO) device operably coupled to the first tubular member.

In various aspects, a kit may be provided. The kit may include a device as disclosed herein, and a controller. The kit may include a blood pump. The kit may include an extracorporeal membrane oxygenation (ECMO) device.

In various aspects, a device for detecting a location of a mixing cloud in a vessel of a subject may be provided. The device may include a tubular member defining a plurality of lumens, including a first, second, and third lumen. The first lumen may be configured to allow blood to flow from a proximal end of the first tubular member to a distal end of the first tubular member. The second lumen may be configured to allow a second tubular member to be slidably received by the second lumen and draw a blood sample at a first location a first axial distance from the proximal end of the first tubular member. The third lumen may be configured to allow a third tubular member to be slidably received by the third lumen and draw a blood sample at a second location at a second axial distance from the proximal end of the first tubular member, the second axial distance being different from the first axial distance.

In various aspects, a device for detecting a location of a mixing cloud in a vessel of a subject. The device may include a tubular member defining a plurality of lumens, including a first, second, and third lumen. The first lumen may be configured to allow blood to flow from a proximal end of the first tubular member to a distal end of the first tubular member. The second lumen may be configured to allow a first pressure sensor to be operably connected to a controller through the second lumen, the first pressure sensor being positioned at or on an external surface of the first tubular member at a first location a first axial distance from the proximal end of the first tubular member. The third lumen may be configured to allow a second pressure sensor to be operably connected to a controller through the third lumen, the second pressure sensor being positioned at or on the external surface of the first tubular member at a second location a second axial distance from the proximal end of the first tubular member, the second axial distance being different from the first axial distance.

### BRIEF DESCRIPTION OF FIGURES

The accompanying drawings, which are incorporated in and constitute a part of this specification, illustrate embodiments of the present invention and, together with a general description of the invention given above, and the detailed description of the embodiments given below, serve to explain the principles of the present invention.
Figure 1 is an illustration of a mixing cloud within a blood vessel.
Figure 2 is a flowchart illustrating an embodiment of a method.
Figure 3A is a cross-sectional side view of a simplified tubular member.
Figure 3B is a cross-sectional end view of a simplified tubular member.
Figure 4 is an illustration of a tubular member with a hub.
Figure 5A is a side view illustration of a tubular member with a pressure sensor.
Figures 5B and 5C are top view illustrations of pressure sensor arrangements.
Figure 5D is a side view illustration of a tubular member with a pressure sensor.
Figure 6 is an illustration of a tubular member within a blood vessel.
Figures 7A and 7B are side view illustrations of blood flowing past a pressure sensor in a first orientation (7A) and a second orientation (7B).
Figure 8 is an illustration of a blood pump introduced into a subject.
Figure 9 is an illustration of a blood pump and an ECMO cannula introduced into a subject.

It should be understood that the appended drawings are not necessarily to scale, presenting a somewhat simplified representation of various features illustrative of the basic principles of the invention. The specific design features of the sequence of operations as disclosed herein, including, for example, specific dimensions, orientations, locations, and shapes of various illustrated components, will be determined in part by the particular intended application and use environment. Certain features of the illustrated embodiments have been enlarged or distorted relative to others to facilitate visualization and clear understanding. In particular, thin features may be thickened, for example, for clarity or illustration.

### DETAILED DESCRIPTION

The following description and drawings merely illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be only for illustrative purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or, unless otherwise indicated (e.g., "or else" or "or in the alternative"). Also, the various embodiments described herein are not necessarily mutually exclusive, as some embodiments can be combined with one or more other embodiments to form new embodiments.

The numerous innovative teachings of the present application will be described with particular reference to the presently preferred exemplary embodiments. However, it should be understood that this class of embodiments provides only a few examples of the many advantageous uses of the innovative teachings herein. In general, statements made in the specification of the present application do not necessarily limit any of the various claimed inventions. Moreover, some statements may apply to some inventive features but not to others. Those skilled in the art and informed by the teachings herein will realize that the invention is also applicable to various other technical areas or embodiments.

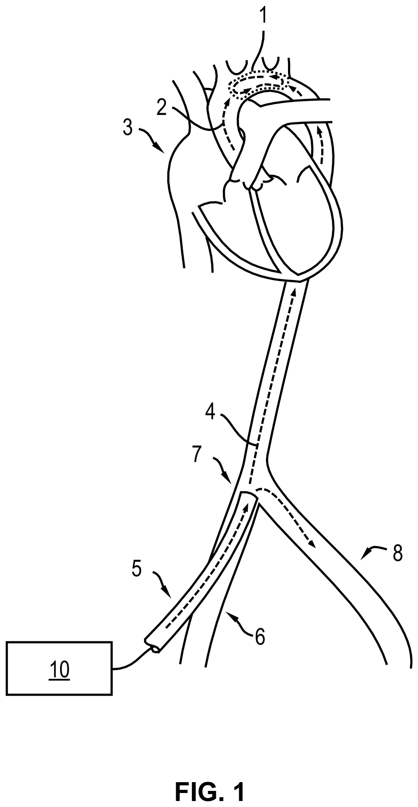

Referring to FIG. 1, a mixing cloud **1** occurs when a patient's native blood flow **2** (flowing in a first direction, e.g., away from the heart **3)** meets with flow **4** from an ECMO device **10** (e.g., through a lumen of a tubular member **5** inserted into a femoral artery **6,** where the tubular member is coupled (such as removably coupled) to the ECMO device). flowing in an opposite direction (*e.g.,* towards the heart) and the two flows "mix". Note, in some embodiment, the distal end **7** of the tubular member delivering oxygenated blood from the ECMO device may be inserted some distance away from the heart, and there may be one or more branches between the distal end of the tubular member and the heart. This is shown in FIG. 1 as a second vessel **8** through which the oxygenated blood from the ECMO device is delivered.

Because the point within the blood vessel that the mixing occurs is a balance between the force of flow generated by the ECMO device, and the force of flow generated by the patient's heart, conceptually a medical professional can determine the degree of recovery of the patient based on the location of the mixing cloud.

For example, in VA-ECMO, the ECMO blood flow infused into the femoral artery is in a direction that is retrograde to the native blood flow. When the heart recovers a pulsatility and the aortic valve begins to open, fully saturated blood from the ECMO mixes with the blood ejected from the native ventricle in the aorta and create a mixing cloud. The location of this mixing cloud depends on the amount of ECMO support provided and the degree of left ventricular ejection. The mixing cloud moves proximally in the aorta when there is poor left ventricular ejection function and/or when the ECMO flow is increased. On the contrary, the mixing cloud moves distally in the aorta when the ventricle recovers and/or the ECMO flow is decreased. If the pulmonary function is impaired, the typical VA ECMO flow rate (80% of full cardiac output) can result in desaturated blood from the left ventricle perfusing the aortic arch, the brain, and coronary arteries, and fully saturated infusion blood perfusing the lower body. The patient's head appears blue, whereas the lower extremities appear pink.

Knowing where the mixing cloud is in the aorta is extremely helpful from a clinician's perspective because it helps indicate how the patient is recovering. When weaning a patient from an ECMO circuit, the location of the mixing cloud is monitored and as the ECMO system flow is reduced, the mixing cloud is expected to move downward toward the aortic bifurcation. Additionally, if the patients are suffering from an oxygenation issue in their lungs, but their left heart is strong, the mixing cloud may actually be far down in the aorta because of the strength of the native heart. In this cases, the patient's great arteries extending off the aortic bifurcation will not get the intended oxygenated blood.

Unfortunately, there are few conventional methods to accurately estimate where the ECMO mixing cloud is. The current leading method is an evaluation of blood oxygen saturation levels from blood drawn from easily accessible parts of the body (femoral artery, radial artery, brachial artery), remote from where the mixing could occur. However, the accuracies associated with these methods are low. Further, these method does not give physicians an exact location or an ability to track the mixing cloud.

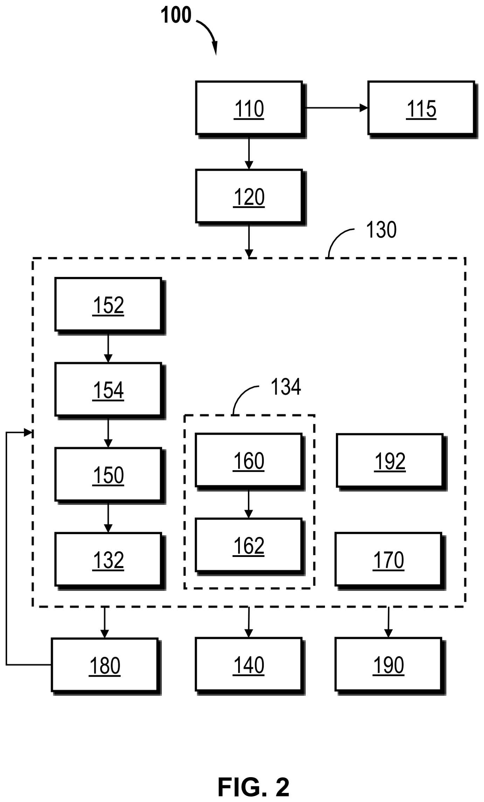

In various aspects, a method for detecting a location of a mixing cloud in a vessel (such as an aorta) of a subject may be provided. Referring to FIG. 2, a method **100** may include positioning **110** a first tubular member within the vessel.

The method may also include locking **115** the first tubular member into a fixed position after positioning the first tubular member within the vessel. This may be done in a variety of ways known to those skilled in the art, including, *e.g.*, using suture pads operably coupled to the tubular member, tightening a Tuohy Borst valve, etc.

The method may include flowing **120** blood through the first lumen from the proximal end to the distal end of either the first tubular member. The first tubular member may include, *e.g*., an extracorporeal membrane oxygenation (ECMO) cannula. The flow may be in in a direction retrograde to a direction blood is natively flowing in the vessel. *See* FIG. 1.

The method may include determining **130** a plurality of blood-related characteristics of blood flowing in the vessel. The method may then include estimating **140** a location of the mixing cloud based on the measured blood-related characteristics.

The measured characteristics may include a plurality of arterial blood oxygen saturation values and/or a plurality of pressure values. That is, in some embodiments, the determining step may include measuring **132** a plurality of arterial blood oxygen saturation values and/or measuring **134** a plurality of pressure values. In some embodiments, only arterial blood oxygen saturation values are measured. In some embodiments, only pressure values are measured. In some embodiments, both are measured. Each saturation value may be determined from blood acquired at a different axial position along the first tubular member. Each pressure value may be determined from a pressure sensor of a plurality of pressure sensors positioned at or on different axial positions along an exterior surface of the first tubular member.

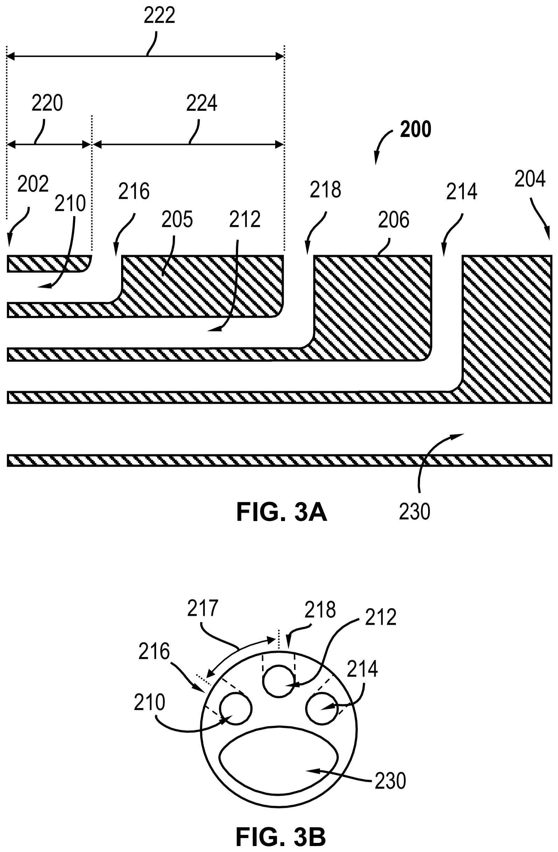

Referring to FIGS. 3A and 3B, the first tubular member **200** may define a first plurality of lumens (*e.g.*, first lumen **210,** second lumen **212,** and third lumen **214).**

Each lumen of the first plurality of lumens may extend from the proximal end **202** towards the distal end **204** but exiting the body **205** of the tubular member at an external surface **206** at different axial positions along the length of the first tubular member as compared to other lumens of the first plurality of lumens. For example, as seen in FIG. 3A, the first lumen **210** may form a first opening **216** at the external surface of the tubular member, and the second lumen **212** may form a second opening **218.** The first opening may begin at a first distance **220** from the proximal end, and the second opening may begin at a second distance **222** from the proximal end. In some embodiments, the axial distance **224** between one opening and an adjacent opening may be fixed. In some embodiments, the axial distance **224** may vary. In some embodiments, the distance from the proximal end to the first opening may be larger than each of the axial distances between adjacent openings.

In some embodiments, the device may have 3 or more lumen and openings. In some embodiments, the device may have 4 or more lumen and openings. In some embodiments, the device may have 5 or more lumen and openings. In some embodiments, the device may have 6 or more lumen and openings.

Referring to FIG. 3B, in some embodiments, the openings may circumferential separated (e.g., by a distance **217,** shown as being a circumferential distance from a centerline of one opening to the centerline of a different opening). Some or all of the openings may be circumferentially separated in addition to being axially separated. Note that the exact end cross-sectional view may vary.

In embodiments where there is circumferential spacing, inherently the distance **217** will always be π*d*/2 or less for a tubular member with a circular cross-section, where d is the outer circumference of the tubular member. However, in some embodiments, the distance may be a distance of π*d*/4 or less. In other embodiments, the distance may be π*d*/2. In some embodiments, the circumferential spacing between one opening and an adjacent opening may be fixed. In some embodiments, the circumferential spacing between one opening and an adjacent opening may vary. In some embodiments, the openings may only be axially separated but not circumferentially separated.

In some embodiments, some or all of the plurality of lumens in the tubular member may be configured to transport blood in a proximal direction.

In some embodiments, the tubular member may be configured to be slidably disposed through an ECMO cannula. In some cases, the tubular member may be configured as an ECMO cannula. For example, in some embodiments, the tubular member may include a lumen **230** that extends from proximal end to a distal end. In some embodiments, this lumen may be configured to transport blood in a distal direction. In some embodiments, this lumen may be configured, *e.g.*, to have a drive shaft disposed within it.

Referring to FIG. 2, in such cases, the method may include drawing **150** a plurality of blood samples, each of which may be drawn through one or more of the plurality of lumens (*e.g*., lumens **210, 212, 214** in FIG. 3A). The plurality of blood-related characteristics may be determined by, *e.g.*, measuring at least the arterial blood oxygen saturation values of each blood sample. In some embodiments, each blood sample may be drawn simultaneously. In some embodiments, each blood sample may be drawn sequentially. In some embodiments, each blood sample may be drawn at a time controlled by at least one processor.

The method may include providing **152** a plurality of blood-drawing tubular members. Each blood-drawing tubular member may have a blood-drawing lumen extending therethrough. Each blood-drawing tubular member may have an outer diameter that is less than an inner diameter of one or more of the plurality of lumens in the first tubular member.

The method may include inserting **154** at least one blood-drawing tubular member at least partially into the plurality of lumens, to allow blood to be drawn through the blood-drawing lumen of the blood-drawing tubular member.

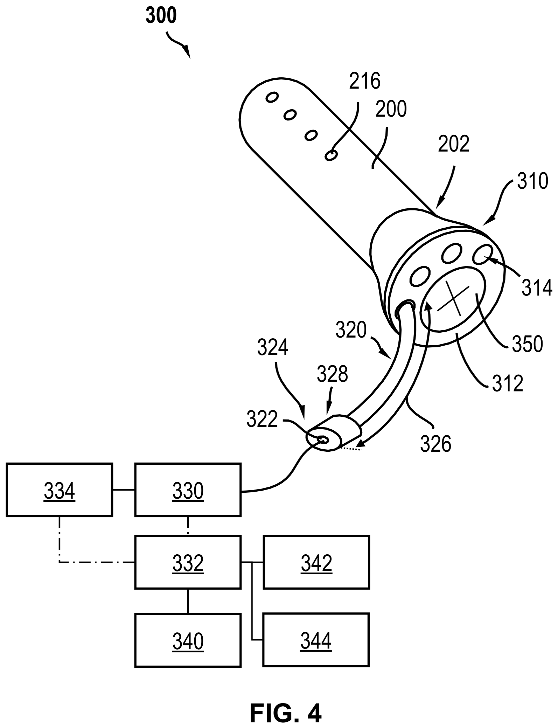

Referring to FIG. 4, in some embodiments, in a system **300,** a hub **310** may be coupled to a proximal end **202** of the first tubular member **200.** The hub may have a plurality of openings **312** on a proximal surface **314** of the hub, each of the openings in the hub being operably coupled to a different lumen in the first tubular member, and thus operably coupled to the opening on the external surface associated with the lumen. For example, a first opening **312** in the hub may be operably coupled to a first opening **216** in the first tubular member.

Each blood-drawing tubular member **320** may be operably coupled to the hub. In some embodiments, each blood-drawing tubular member may be operably coupled to the hub at or near a proximal end **322** of each blood-drawing tubular member. For example, in some embodiments, the distal ends may be a distance **326** (along the axis of the tubular member) *b* that may be, *e.g., b* ≤ 6 inches, b ≤ 5 inches, *b* ≤ 4 inches, *b* ≤ 3 inches, *b* ≤ 2 inches, or *b* ≤ 1 inch.

In some embodiments, the blood-drawing lumen **322** in each blood-drawing tubular member may be operably coupled to, *e.g.,* one or more devices. For example, in some embodiments, the blood-drawing lumen may be coupled to a pump **330,** which may be controlled by, *e.g.,* one or more processors **332.** The one or more processors may be operably coupled to one or more sensors **334** (*e.g.,* a blood gas sensor) that are configured to analyze blood drawn using, *e.g.,* the pump. In some embodiments, blood may be drawn manually.

In some embodiments, each blood-drawing tubular member may include a coupling, fitting, or adapter **328.** The coupling, fitting, or adapter may be configured for allowing the blood-drawing tubular member to be removably coupled to the pump. The coupling, fitting, or adapter may be configured to allow for direct blood draws through the coupling, fitting, or adapter.

In some embodiments, some or all of the plurality of lumens in the first tubular member may include an electrical or optical communication conduit coupled to a pressure sensor of the plurality of pressure sensors. Referring to FIG. 2, the step of measuring **134** a plurality of pressure values may include receiving **160** information from the plurality of pressure sensors. The plurality of blood-related characteristics may be determined by determining **162** a pressure value based on the received information from each pressure sensor.

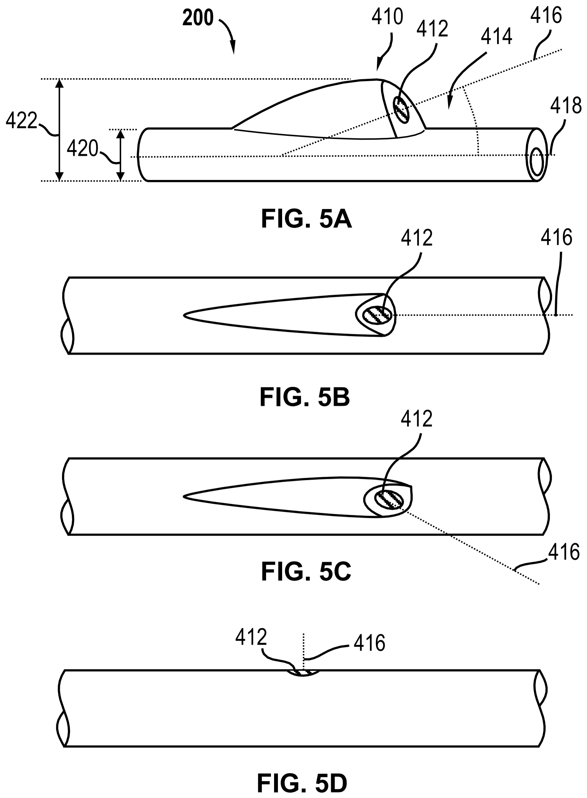

Referring to FIG. 5A, each pressure sensor **412** may positioned within a separate pressure sensor portion **410** of the first tubular member. The sensor portion may have an effective diameter **422** that is larger than an effective diameter **420** in another portion of the first tubular member.

The sensor may be oriented in a predetermined direction, and a line **416** may exist that passes through a center of the pressure sensor and is oriented in the same direction the sensor is oriented.

Some or all of the sensors may be oriented in configurations that are non-orthogonal with the central axis.

Referring to FIGS. 5A and 5B, in some embodiments, the line **416** and the central axis **418** of the first tubular member are coplanar, and an angle **414** may exist formed by the line and the central axis that may be acute (less than 90 degrees). The acute angle indicates which direction the sensor is facing (using FIG. 5A as an example, the acute angle faces to the right, so the sensor is facing to the right) In some embodiments, that angle may be less than 70 degrees. In some embodiments, that angle may be less than 60 degrees. In some embodiments, that angle may be less than 45 degrees. In some embodiments, that angle may be less than 30 degrees. In some embodiments, that angle may be less than 20 degrees. In some embodiments, the angle may be 0 degrees. In some embodiments, the angle may be greater than 0 degrees.

Referring to FIG. 5C, in some embodiments, the line **416** may be non-coplanar with the central axis (i.e., a skew line).

In various embodiments, one or more sensors may be oriented in a configuration that is orthogonal with the central axis. Referring to FIG. 5D, in some embodiments, the line **416** may be orthogonal to the central axis.

In some embodiments, each pressure sensor may be oriented in a non-orthogonal direction relative to the axis of the first tubular member. In some embodiments, some pressure sensors may be oriented in a non-orthogonal direction relative to the axis of the first tubular member, and some may be oriented in an orthogonal direction. In some embodiments, all pressure sensors of the plurality of pressure sensors are oriented towards the distal end or the proximal end of the first tubular member (e.g., with sensors such as those seen in FIGS. 5A and 5B, where all sensors face either proximally or distally. In some embodiments, at least one sensor faces distally, and one sensor faces proximally.

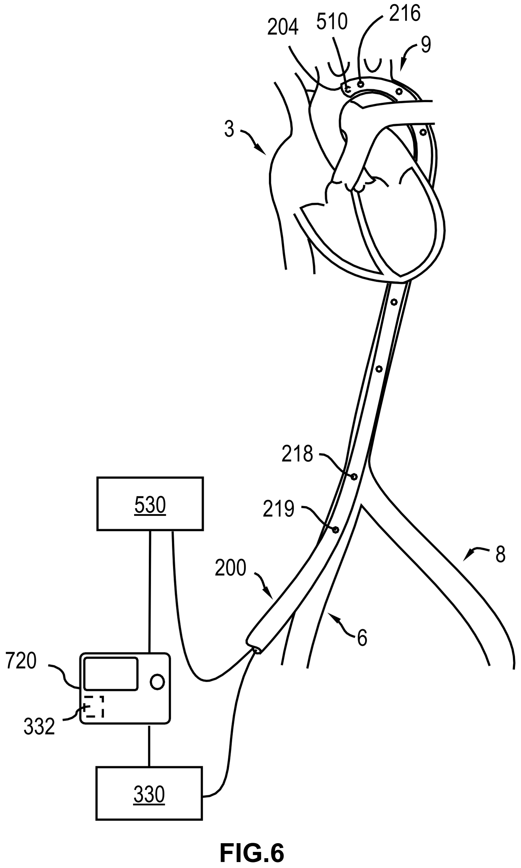

Referring to FIG. 6, in some embodiments, the first tubular member **200** may extend through a femoral artery **6,** and may extend at least partially through an aortic arch **9** (*e.g.*, distal end **204** may be in the aortic arch). In some embodiments, at least one opening **216** may be disposed in the aortic arch. In some embodiments, at least one opening **218** may be located around a branch (*e.g*., where blood may start to flow down second vessel **8).** In some embodiments, at least one opening **219** may be located proximal to the branch.

In some embodiments, the location of the mixing cloud may be estimated by determining where oxygenated blood being supplied by the ECMO device passing through the first lumen is mixing with unoxygenated blood from the subject's heart based on the oxygen content of the blood pulled from each location in the aorta.

For example, if an opening is positioned one either side of a mixing cloud, the one opening will measure relatively low oxygenation while the other will measure relatively high oxygenation. Based on the relative values and the known configuration of the openings, a position of the mixing cloud can be estimated.

In some embodiments, if the arterial blood oxygen saturation value of the incoming blood from the ECMO device is known, the gradient of measured arterial blood oxygen saturation values (e.g., the values determined from blood drawn from consecutive openings) can be used to estimate the mixing cloud location.

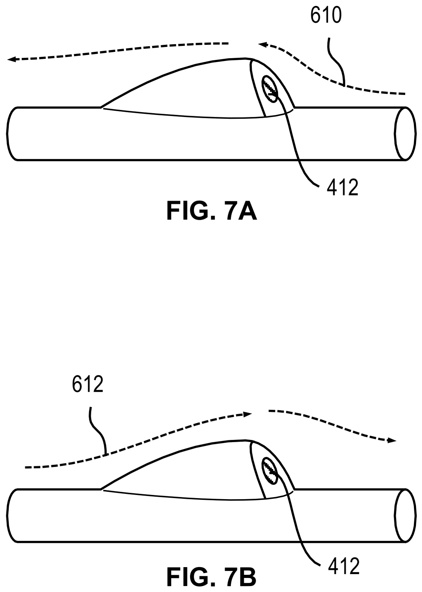

In some embodiments, the location of the mixing cloud may be estimated by determining a direction of flow at each pressure sensor location based on the plurality of pressure values and estimating a location of an inflection point in the direction of flow along the length of the first tubular member based on the determined directions of flow. The pressure of blood flowing in a direction **610** substantially counter to the direction the sensor **412** is oriented (FIG. 7A, sensor is facing the upstream direction of blood flow) will be different (*e.g.*, relatively higher) than the pressure of blood flowing in the substantially the same direction **612** as the direction the sensor is oriented (FIG. 7B, sensor is facing the downstream direction of blood flow). Referring to FIG. 1, a position of the mixing cloud can be estimated by estimating where the direction of flow changes (*e.g.*, reverses) based on the various pressure readings.

As will be understood, these two approaches can be combined.

Referring to FIG. 2, the method may include measuring **170** an acceleration of the first tubular member. The plurality of blood-related characteristics may be determined based at least partially on the measured acceleration. Referring to FIG. 6, in some embodiments, the tubular member may include an accelerometer **510.** In some embodiments, this may be positioned at or near the distal end **204.** In some embodiments, multiple accelerometers may be used. In some embodiments, an accelerometer may be positioned at a proximal end or in a hub coupled to the tubular member.

Referring to FIG. 2, the method may include adjusting **180** the position of the first tubular member after determining the plurality of blood-related characteristics of blood flowing in the vessel.

Referring to FIG. 4, in some embodiments, the processor(s) **332** may be operably coupled to, *e.g.,* a memory **340,** a non-transitory computer-readable storage device **342,** and a display **344.**

The method may include generating **190** a graphical display. In some embodiments, the method may include graphically displaying the estimated location of the mixing cloud. In some embodiments, the method may include graphically displaying a location of the first tubular member and/or a blood pump.

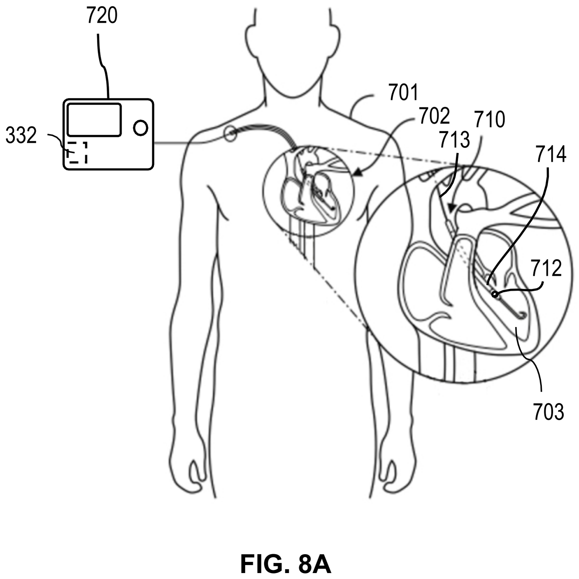

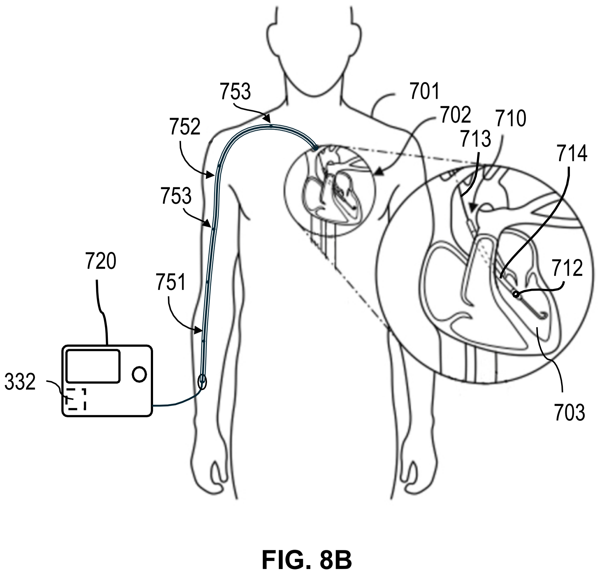

Referring to FIG. 8, in some embodiments, it can be seen that a hemodynamic support device **710** configured to be positioned in a first area **703** of a body of a subject **701.** The hemodynamic support device may include, *e.g*., a blood pump **714,** which may be coupled to a distal end of a catheter **713.** In FIG. 1, the blood pump is shown as being placed in the heart **702,** and specifically in the left heart.

The system may include at least one processor, such as processor **332.** The processor may be operably coupled to the hemodynamic support device, either via a wired or wireless connection. In some embodiments, the hemodynamic support device may be removably coupled via one or more wires to a controller **720.** The controller may include a processor **332** operably coupled to a memory, a non-transitory computer-readable storage medium, a display, and/or one or more physical controls, such as buttons or knobs. As will be understood, the non-transitory computer-readable storage medium may contain instructions that, when executed by the processor, configured the processor to perform certain steps.

The hemodynamic support device may include one or more sensors **712.** Such sensors may be any appropriate sensor for the intended data to be collected, and may include, *e.g.*, electrodes, optical sensors and/or pressure sensors.

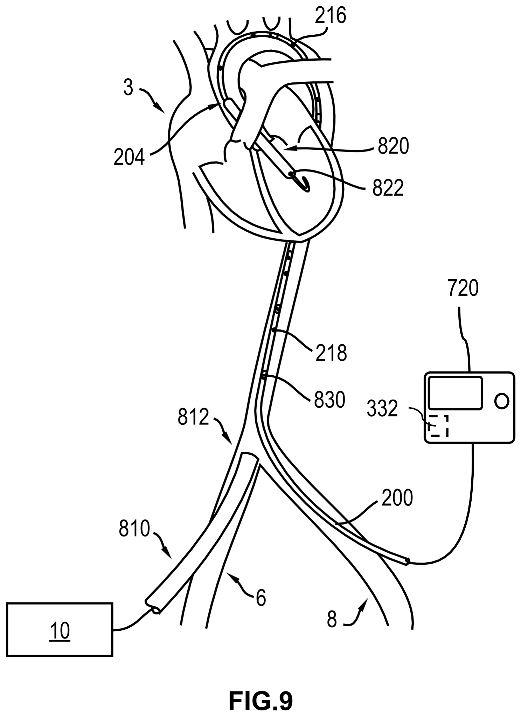

Referring to FIGS. 2 and 9, the method may include receiving **192** information (*e.g*., at one or more processor(s) 332 in a controller 720) from at least one sensor **822** operably coupled to a blood pump **820** positioned in a heart **3.** As seen in FIG. 9, in some embodiments, a distal end **204** of the first tubular member **200** may be operably coupled to a blood pump **820.** The blood pump may have one or more sensors **822** in or on the blood pump, which can provide information to the processor(s), in addition to, *e.g*., pressure sensors in the first tubular member and/or blood-gas sensors from blood drawn through openings **216, 218** in the first tubular member. In FIG. 9, it can be seen that a distal end **812** of the ECMO cannula **810** (or another tubular member with a lumen extending from a proximal end to a distal end of the tubular member) may positioned in a first vessel **6** at or below the branch for the vessel **8** through which the first tubular member connects. In some embodiments, blood may flow through this second tubular member, while the first tubular member is used to determine the positioning of the mixing cloud.

In some embodiments, estimating the location of the mixing cloud may be based on the blood-related characteristics and the information received from the at least one sensor operably coupled to the blood pump. In some embodiments, the location of the mixing cloud may be estimated relative to a position of the blood pump.

In various aspects, a device for detecting a location of a mixing cloud in a vessel of a subject may be provided. Referring to FIGS. 3A and 3B, the device may include a tubular member **200** with a body **205** defining a first lumen **230** extending from a proximal end **202** to a distal end **204** of the first tubular member. The device may include a first plurality of lumens **210, 212** where each lumen of the first plurality of lumens extends from the proximal end to an external surface **206** of the first tubular member at a different axial position along the first tubular member as compared to other lumens of the first plurality of lumens (*e.g.*, different distances **220, 222).**

The tubular member may include one or more biocompatible polymers or metals.

Each lumen of the first plurality of lumens may be configured to allow blood to be drawn from a subject and through the proximal end of the first tubular member.

Referring to FIGS. 5A-5D, the device may include a plurality of pressure sensors, each pressure sensor **412** being positioned at the distal end of one of the first plurality of lumens.

The first tubular member may have a first circumference **420** at the proximal end, and a second circumference **422** at the distal ends of each of the first plurality of lumens, the second circumference being greater than the first circumference.

Referring to FIG. 9, the device may include at least one fluoroscopic marker **830** and/or radiopaque material. This may include a metal band and/or a filler polymer placed on the first tubular member at predetermined locations.

In various aspects, a system may be provided. Referring to FIG. 6, the system may include a device as disclosed herein, and a controller **720** (which may include one or more processor(s) **332)** operably coupled to the device and at least one pump (here, ECMO pump **530** and a blood-draw pump **330** are shown) configured to cause blood to flow through the device.

The controller may include at least one processor **332** configured to measure an arterial blood oxygen saturation value (*e.g.*, from a blood-gas sensor **334,** see FIG. 4) of each blood sample of a plurality of blood samples received from the device, correlate each arterial blood oxygen saturation value with an axial location along the first tubular member, and estimate a location of the mixing cloud by calculating an axial location at which a theoretical arterial blood oxygen saturation value, based on the measured arterial blood oxygen saturation values and the correlation, would be expected to equal a predetermined value or range of values.

The controller may include at least one processor configured to determine a pressure value associated with each pressure sensor reading received from a plurality of pressure sensors, correlate each pressure value with an axial location along the first tubular member, and estimate a location of the mixing cloud by calculating an axial location at which a theoretical pressure value, based on the determined pressure values and the correlation, would be expected to equal a predetermined value or range of values.

The system may include an extracorporeal membrane oxygenation (ECMO) device operably coupled to the first tubular member. See, *e.g.,* FIGS. 4 and 6, ECMO pump **530** may be operably coupled to hub **310,** where cannula may pass through a hemostasis valve **350** to allow oxygenated blood to enter the tubular member through the hub.

In various aspects, a kit may be provided. The kit may include a device as disclosed herein, and a controller. The kit may include a blood pump. The kit may include an extracorporeal membrane oxygenation (ECMO) device.

In various aspects, a device for detecting a location of a mixing cloud in a vessel of a subject may be provided. The device may include a tubular member defining a plurality of lumens, including a first, second, and third lumen. See FIG. 3A. The first lumen may be configured to allow blood to flow from a proximal end of the first tubular member to a distal end of the first tubular member. The second lumen may be configured to allow a second tubular member to be slidably received by the second lumen and draw a blood sample at a first location a first axial distance from the proximal end of the first tubular member. The third lumen may be configured to allow a third tubular member to be slidably received by the third lumen and draw a blood sample at a second location at a second axial distance from the proximal end of the first tubular member, the second axial distance being different from the first axial distance.

In various aspects, a device for detecting a location of a mixing cloud in a vessel of a subject. The device may include a tubular member defining a plurality of lumens, including a first, second, and third lumen. See FIG. 3A. The first lumen may be configured to allow blood to flow from a proximal end of the first tubular member to a distal end of the first tubular member. The second lumen may be configured to allow a first pressure sensor to be operably connected to a controller through the second lumen, the first pressure sensor being positioned at or on an external surface of the first tubular member at a first location a first axial distance from the proximal end of the first tubular member. The third lumen may be configured to allow a second pressure sensor to be operably connected to a controller through the third lumen, the second pressure sensor being positioned at or on the external surface of the first tubular member at a second location a second axial distance from the proximal end of the first tubular member, the second axial distance being different from the first axial distance.

Various modifications may be made to the systems, methods, apparatus, mechanisms, techniques and portions thereof described herein with respect to the various figures, such modifications being contemplated as being within the scope of the invention. For example, while a specific order of steps or arrangement of functional elements is presented in the various embodiments described herein, various other orders/arrangements of steps or functional elements may be utilized within the context of the various embodiments. Further, while modifications to embodiments may be discussed individually, various embodiments may use multiple modifications contemporaneously or in sequence, compound modifications and the like.

Specific embodiments can be understood as described below.

In a first embodiment, a method for detecting a location of a mixing cloud in a vessel of a subject may be provided, where the method includes: (i) positioning a first tubular member within the vessel; (ii) flowing blood through a first lumen from a proximal end to a distal end of either the first tubular member or a second tubular member in a direction retrograde to a direction blood is natively flowing in the vessel; (iii) determining a plurality of blood-related characteristics of blood flowing in the vessel, including: (a) a plurality of arterial blood oxygen saturation values, each determined from blood acquired at a different axial position along the first tubular member, (b) a plurality of pressure values, each determined from a pressure sensor of a plurality of pressure sensors positioned at or on different axial positions along an exterior surface of the first tubular member, or (c) a combination thereof; and (iv) estimating a location of the mixing cloud based on the blood-related characteristics.

In a second embodiment, based on the first embodiment, the first tubular member or second tubular member comprises an extracorporeal membrane oxygenation (ECMO) cannula.

In a third embodiment, based on the first or second embodiments, the first tubular member defines a first plurality of lumens each lumen of the first plurality of lumens extending from the proximal end to an external surface of the first tubular member at a different axial position along the first tubular member as compared to other lumens of the first plurality of lumens.

In a fourth embodiment, based on the third embodiment, each lumen of the first plurality of lumens is configured to transport blood in a proximal direction.

In a fifth embodiment, based on the fourth embodiment, the method further includes: (i) drawing a plurality of blood samples, each drawn through one lumen of the first plurality of lumens; and (ii) determining the plurality of blood-related characteristics by measuring the arterial blood oxygen saturation values of each blood sample.

In a sixth embodiment, based on the fifth embodiment, each blood sample is drawn simultaneously.

In a seventh embodiment, based on the fifth embodiment, each blood sample is drawn sequentially.

In an eighth embodiment, based on the fifth embodiment, each blood sample is drawn at a time controlled by at least one processor.

In a ninth embodiment, based on any of the third through the eighth embodiments, the method further includes: (i) providing a plurality of third tubular members, each third tubular member having a lumen extending therethrough; and (ii) inserting one tubular member of the plurality of third tubular members at least partially into one of the first plurality of lumens, to allow blood to be drawn through the lumen of the third tubular member.

In a tenth embodiment, based on the ninth embodiment, the plurality of third tubular members are operably coupled to a hub at or near a proximal end of each third tubular member.

In an eleventh embodiment, based on the third through the tenth embodiments, each lumen of the first plurality of lumens includes an electrical or optical communication conduit coupled to a pressure sensor of the plurality of pressure sensors.

In a twelfth embodiment, based on the eleventh embodiment, the method further includes: (i) receiving information from the plurality of pressure sensors; and (ii) determining the plurality of blood-related characteristics by determining a pressure value based on the received information from each pressure sensor.

In a thirteenth embodiment, based on any of the first through twelfth embodiments, each pressure sensor of the plurality of pressure sensors is oriented in a non-orthogonal direction relative to an axis of the first tubular member.

In a fourteenth embodiment, based on the thirteenth embodiment, all pressure sensors of the plurality of pressure sensors are oriented towards the distal end or the proximal end of the first tubular member.

In a fifteenth embodiment, based on any of the first through fourteenth embodiments, the vessel is an aorta.

In a sixteenth embodiment, based on the fifteenth embodiment, the first tubular member extends through a femoral artery.

In a seventeenth embodiment, based on the fifteenth embodiment, the first tubular member extends at least partially through an aortic arch.

In an eighteenth embodiment, based on any one of the first through seventeenth embodiments, the location of the mixing cloud is estimated by determining where oxygenated blood passing through the first lumen is mixing with unoxygenated blood from a heart of the subject based on oxygen content of the blood pulled from each location in an aorta of the subject.

In a nineteenth embodiment, based on any one of the first through seventeenth embodiments, the location of the mixing cloud is estimated by determining a direction of flow at each pressure sensor location based on the plurality of pressure values and estimating a location of an inflection point in the direction of flow along a length of the first tubular member based on the determined directions of flow.

In a twentieth embodiment, based on any one of the first through nineteenth embodiments, the method further includes locking the first tubular member into a fixed position after positioning the first tubular member within the vessel.

In a twenty-first embodiment, based on any one of the first through twentieth embodiments, the method further includes adjusting the position of the first tubular member after determining the plurality of blood-related characteristics of blood flowing in the vessel.

In a twentieth-second embodiment, based on any one of the first through twenty-first embodiments, the method further includes measuring an acceleration of the first tubular member.

In a twentieth-third embodiment, based on the twenty-second embodiment, determining the plurality of blood-related characteristics is based on the measured acceleration.

In a twentieth-fourth embodiment, based on any one of the first through twenty-third embodiment, the method further includes graphically displaying the estimated location of the mixing cloud.

In a twentieth-fifth embodiment, based on the twenty-fourth embodiment, the method further includes graphically displaying a location of the first tubular member and/or a blood pump.

In a twentieth-sixth embodiment, based on any one of the first through twenty-fifth embodiment, the method further includes receiving information from at least one sensor operably coupled to a blood pump positioned in a heart.

In a twentieth-seventh embodiment, based on the twenty-sixth embodiment, estimating the location of the mixing cloud is based on the blood-related characteristics and the information received from the at least one sensor operably coupled to the blood pump.

In a twentieth-eighth embodiment, based on the twenty-sixth embodiment, the location of the mixing cloud is estimated relative to a position of the blood pump.

In a twenty-ninth embodiment, a device for detecting a location of a mixing cloud in a vessel of a subject may be provided, the device including: (i) a tubular member defining a first lumen extending from a proximal end to a distal end of the first tubular member; and (ii) a first plurality of lumens, where each lumen of the first plurality of lumens extends from the proximal end to an external surface of the first tubular member at a different axial position along the first tubular member as compared to other lumens of the first plurality of lumens.

In a thirtieth embodiment, based on the twenty-ninth embodiment, each lumen of the first plurality of lumens is configured to allow blood to be drawn from a subject and through the proximal end of the first tubular member.

In a thirty-first embodiment, based on the twenty-ninth or thirtieth embodiment, the first tubular member has a first circumference at the proximal end, and a second circumference at the distal ends of each of the first plurality of lumens, the second circumference being greater than the first circumference.

In a thirty-second embodiment, based on any one of the twenty-ninth through the thirty-first embodiments, the device may include a plurality of pressure sensors, each pressure sensor being positioned at the distal end of one of the first plurality of lumens.

In a thirty-third embodiment, based on the thirty second embodiment, each pressure sensor of the plurality of pressure sensors is oriented in a non-orthogonal direction relative to an axis of the first tubular member.

In a thirty-fourth embodiment, based on the thirty-second embodiment, all pressure sensors of the plurality of pressure sensors are oriented towards the distal end or the proximal end of the first tubular member.

In a thirty-fifth embodiment, based on any one of the twenty-ninth through thirty-fourth embodiment, the device further includes at least one fluoroscopic marker and/or radiopaque material.

In a thirty-sixth embodiment, based on the thirty-fifth embodiment, the at least one fluoroscopic marker and/or radiopaque material includes a metal band and/or a filler polymer placed on the first tubular member at predetermined locations.

In a thirty-seventh embodiment, a system for detecting a location of a mixing cloud in a vessel of a subject may be provided, the system including: (i) a device according to any one of embodiments twenty-nine through thirty-six; and (ii) a controller operably coupled to the device and at least one pump configured to cause blood to flow through the device.

In a thirty-eighth embodiment, based on the thirty-seventh embodiment, the controller includes at least one processor configured to (collectively): (i) measure an arterial blood oxygen saturation value of each blood sample of a plurality of blood samples received from the device; (ii) correlate each arterial blood oxygen saturation value with an axial location along the first tubular member; and (iii) estimate a location of the mixing cloud by calculating an axial location at which a theoretical arterial blood oxygen saturation value, based on the measured arterial blood oxygen saturation values and the correlation, would be expected to equal a predetermined value or range of values.

In a thirty-ninth embodiment, based on the thirty-seventh or thirty-eighth embodiment, the controller includes at least one processor configured to (collectively): (i) determine a pressure value associated with each pressure sensor reading received from a plurality of pressure sensors; (ii) correlate each pressure value with an axial location along the first tubular member; and (iii) estimate a location of the mixing cloud by calculating an axial location at which a theoretical pressure value, based on the determined pressure values and the correlation, would be expected to equal a predetermined value or range of values.

In a fortieth embodiment, based on any one of the thirty-seventh through thirty-ninth embodiments, the system further includes an extracorporeal membrane oxygenation (ECMO) device operably coupled to the first tubular member.

In a forty-first embodiment, a kit may be provided that includes: (i) a device according to any one of embodiments twenty-nine to thirty-six; and (ii) a controller.

In a forty-second embodiment, based on the forty-first embodiment, the kit further includes a blood pump.

In a forty-third embodiment, based on the forty-first or forty-second embodiment, the kit may further include an extracorporeal membrane oxygenation (ECMO) device.

In a forty-fourth embodiment, a device for detecting a location of a mixing cloud in a vessel of a subject may be provided, the device comprising a tubular member defining a plurality of lumens, including: (i) a first lumen configured to allow blood to flow from a proximal end of the first tubular member to a distal end of the first tubular member; (ii) a second lumen configured to allow a second tubular member to be slidably received by the second lumen and draw a blood sample at a first location a first axial distance from the proximal end of the first tubular member; and (iii) a third lumen configured to allow a third tubular member to be slidably received by the third lumen and draw a blood sample at a second location at a second axial distance from the proximal end of the first tubular member, the second axial distance being different from the first axial distance.

In a forty-fifth embodiment, a device for detecting a location of a mixing cloud in a vessel of a subject may be provided. The device includes a tubular member defining a plurality of lumens, including: (i) a first lumen configured to allow blood to flow from a proximal end of the first tubular member to a distal end of the first tubular member; (ii) a second lumen configured to allow a first pressure sensor to be operably connected to a controller through the second lumen, the first pressure sensor being positioned at or on an external surface of the first tubular member at a first location a first axial distance from the proximal end of the first tubular member; and (iii) a third lumen configured to allow a second pressure sensor to be operably connected to a controller through the third lumen, the second pressure sensor being positioned at or on the external surface of the first tubular member at a second location a second axial distance from the proximal end of the first tubular member, the second axial distance being different from the first axial distance.

Although various embodiments which incorporate the teachings of the present invention have been shown and described in detail herein, those skilled in the art can readily devise many other varied embodiments that still incorporate these teachings. Thus, while the foregoing is directed to various embodiments of the present invention, other and further embodiments of the invention may be devised without departing from the basic scope thereof. As such, the appropriate scope of the invention is to be determined according to the claims.

## Claims

1. A method for detecting a location of a mixing cloud in a vessel of a subject, comprising:
positioning a first tubular member within the vessel;
flowing blood through a first lumen from a proximal end to a distal end of either the first tubular member or a second tubular member in a direction retrograde to a direction blood is natively flowing in the vessel;
determining a plurality of blood-related characteristics of blood flowing in the vessel, including:
a plurality of arterial blood oxygen saturation values, each determined from blood acquired at a different axial position along the first tubular member,
a plurality of pressure values, each determined from a pressure sensor of a plurality of pressure sensors positioned at or on different axial positions along an exterior surface of the first tubular member, or
a combination thereof; and
estimating a location of the mixing cloud based on the blood-related characteristics.

2. The method according to claim 1, wherein the first tubular member or second tubular member comprises an extracorporeal membrane oxygenation (ECMO) cannula.

3. The method according to claim 1 or 2, wherein the first tubular member defines a first plurality of lumens each lumen of the first plurality of lumens extending from the proximal end to an external surface of the first tubular member at a different axial position along the first tubular member as compared to other lumens of the first plurality of lumens.

4. The method according to claim 3, wherein each lumen of the first plurality of lumens is configured to transport blood in a proximal direction.

5. The method according to claim 4, further comprising:
drawing a plurality of blood samples, each drawn through one lumen of the first plurality of lumens; and
determining the plurality of blood-related characteristics by measuring the arterial blood oxygen saturation values of each blood sample.

6. The method according to any one of claims 3-5, further comprising:
providing a plurality of third tubular members, each third tubular member having a lumen extending therethrough; and
inserting one tubular member of the plurality of third tubular members at least partially into one of the first plurality of lumens, to allow blood to be drawn through the lumen of the third tubular member.

7. The method according to any one of claims 3-6, wherein each lumen of the first plurality of lumens includes an electrical or optical communication conduit coupled to a pressure sensor of the plurality of pressure sensors.

8. The method according to claim 7, further comprising:
receiving information from the plurality of pressure sensors; and
determining the plurality of blood-related characteristics by determining a pressure value based on the received information from each pressure sensor.

9. The method according to any one of claims 1-8,
wherein the location of the mixing cloud is estimated by determining where oxygenated blood passing through the first lumen is mixing with unoxygenated blood from a heart of the subject based on oxygen content of the blood pulled from each location in an aorta of the subject, or
wherein the location of the mixing cloud is estimated by determining a direction of flow at each pressure sensor location based on the plurality of pressure values and estimating a location of an inflection point in the direction of flow along a length of the first tubular member based on the determined directions of flow.

10. The method according to any one of claims 1-9,
further comprising locking the first tubular member into a fixed position after positioning the first tubular member within the vessel;
further comprising adjusting the position of the first tubular member after determining the plurality of blood-related characteristics of blood flowing in the vessel; and/or
further comprising measuring an acceleration of the first tubular member.

11. The method according to any one of claims 1-10,
further comprising graphically displaying the estimated location of the mixing cloud; and/or
further comprising receiving information from at least one sensor operably coupled to a blood pump positioned in a heart.

12. The method according to claim 11,
wherein estimating the location of the mixing cloud is based on the blood-related characteristics and the information received from the at least one sensor operably coupled to the blood pump, or
wherein the location of the mixing cloud is estimated relative to a position of the blood pump.

13. A device for detecting a location of a mixing cloud in a vessel of a subject, comprising:
a tubular member defining a first lumen extending from a proximal end to a distal end of the first tubular member; and
a first plurality of lumens, where each lumen of the first plurality of lumens extends from the proximal end to an external surface of the first tubular member at a different axial position along the first tubular member as compared to other lumens of the first plurality of lumens.

14. A system for detecting a location of a mixing cloud in a vessel of a subject, comprising:
a device according to claim 13; and
a controller operably coupled to the device and at least one pump configured to cause blood to flow through the device.

15. A kit, comprising:
a device according to claim 13; and
a controller.
